Europäisches Patentamt

European Patent Office

Office européen des brevets

(19)

(11) Veröffentlichungsnummer : **0 182 176
B1**

(12) **EUROPÄISCHE PATENTSCHRIFT**

(45) Veröffentlichungstag der Patentschrift :
05.04.89

(21) Anmeldenummer : 85113974.1

(22) Anmeldetag : 26.04.83

(60) Veröffentlichungsnummer der früheren Anmeldung
nach Art. 76 EPÜ : 0093378

(51) Int. Cl.⁴ : **A 61 F   2/36**

(54) **Verfahren zum Herstellen von Endoprothesen.**

(30) Priorität : 03.05.82 DE 3216539

(43) Veröffentlichungstag der Anmeldung :
28.05.86 Patentblatt 86/22

(45) Bekanntmachung des Hinweises auf die Patenterteilung : 05.04.89 Patentblatt 89/14

(84) Benannte Vertragsstaaten :
**CH DE FR GB IT LI SE**

(56) Entgegenhaltungen :
EP--A-- 0 093 869
DE--A-- 2 933 229
FR--A-- 1 278 359

(73) Patentinhaber : **Waldemar Link GmbH & Co
Barkhausenweg 10
D-2000 Hamburg 63 (DE)**

(72) Erfinder : **Keller, Arnold
An der Naherfurth 5
D-2061 Keyhude (DE)**

(74) Vertreter : **Glawe, Delfs, Moll & Partner Patentanwälte
Postfach 26 01 62 Liebherrstrasse 20
D-8000 München 26 (DE)**

EP 0 182 176 B1

## Beschreibung

Die Erfindung betrifft ein Verfahren zum Herstellen von Endoprothesen mit in die Markhöhle von Knochen einzusetzenden Halteschäften unterschiedlicher Gestalt, bei dem die Oberflächen von Rohlingen gleicher Gestalt unterschiedlich bearbeitet werden.

Es ist bekannt, Prothesenschäfte zur besseren Anpassung an die individuelle Knochenform spanhebend zu bearbeiten. Diese Bearbeitung erfolgt in der Regel von Hand, weil Steuerungsprogramme für Einzelexemplare nicht zur Verfügung stehen. Da aber die Bearbeitung von Hand teuer und fehleranfällig ist, ist die individuelle Anpassung von Prothesen auf Ausnahmefälle beschränkt, in denen dies wegen ungewöhnlicher Knochengestaltung unumgänglich ist, obwohl eine breitere Anwendung der individuellen Anpassung wünschenswert wäre.

Die spanhebende Oberflächenbearbeitung von Prothesenschäften hat den Nachteil, daß die Oberflächenbeschaffenheit nicht anders sein kann als sie durch die spanhebende Bearbeitung erzielbar ist. Oftmals ist eine andere Oberflächenbeschaffenheit zur besseren Verankerung des Knochengewebes erwünscht, beispielsweise eine porige oder globulare oder durchbrochene Gestalt.

Der Erfindung liegt daher die Aufgabe zugrunde, ein Verfahren der eingangs genannten Art zu schaffen, das die individuelle Bearbeitung der Schaftoberfläche erleichtert und auch mindestens teilweise eine Oberflächenbeschaffenheit ermöglicht, die durch die Bearbeitung nicht erzielbar ist.

Die erfindungsgemäße Lösung besteht darin, daß die Rohlinge mit von einem Schaftkern vorstehenden Rippen hergestellt und die Rippenrücken bearbeitet werden.

Zum einen wird dadurch eine maschinelle Bearbeitung möglich, weil die Bearbeitung jeweils in einzelnen, durch die Rippen vorgegebenen Ebenen erfolgen kann, in denen die Kurvenform der Rippenrücken jeweils besonders bestimmt und dann gesondert auf üblichen Maschinen gefräst wird. Dies wird dadurch ermöglicht, daß keine kontinuierlich durchgehende Oberfläche geschaffen werden muß, sondern die Bearbeitung jeweils auf die voneinander auf Rippenzwischenräume getrennten Rippen beschränkt werden kann.

Zum anderen wird durch die Bearbeitung die Oberflächenbeschaffenheit in den Rippenzwischenräumen nicht geändert. Es besteht daher die Möglichkeit, innerhalb der Rippenzwischenräume eine Oberflächenbeschaffenheit zu wählen, wie sie zur innigen Verbindung mit dem Knochengewebe vorteilhaft erscheint.

Es versteht sich, daß die Querschnittshöhe der Rippen groß genug bemessen werden muß, damit eine Anpassung an unterschiedliche Femurabmessungen durch Abtragung vom Rippenrücken erzielbar ist.

Der Begriff Rippen beschränkt die Querschnittsgestalt der so bezeichneten Prothesenteile nicht. Die Rippen brauchen in ihrer Längsrichtung nicht ununterbrochen ausgebildet zu sein. Vielmehr können von außen nach innen geführte Vertiefungen, Schlitze oder Unterbrechungen zur Beeinflussung ihrer statischen Festigkeit vorteilhaft sein. Zweckmäßigerweise sind auf der dorsalen und der ventralen Seite des proximalen Endabachnitts des Schafts jeweils mindestens zwei Rippen vorgesehen. Insbesondere im endnahen, dickeren Schaftbereich können auch mehr, beispielsweise drei Rippen vorgesehen sein.

Es wird darauf hingewiesen, daß das europäische Patent 0 093 378 Ansprüche enthält, die sich auf eine femorale Hüftgelenkprothese mit an ihrem Schaft vorgesehenen Rippen beziehen.

Die Erfindung wird im folgenden näher unter Bezugnahme auf die Zeichnung erläutert. Darin zeigen :

Fig. 1 einen Längsschnitt in der lateral-medial-Ebene eines Oberachenkelknochens mit eingesetzter Hüftgelenkprothese,

Fig. 2 einen Schnitt derselben Anordnung in der anteriorposterior-Ebene

Fig. 3 eine der Fig. 2 entsprechende Schnittansicht mit geänderter Rippenform und

Fig. 4 einen Horizontalschnitt durch Knochen- und Prothesenschaft nahe dem proximalen Schaftende.

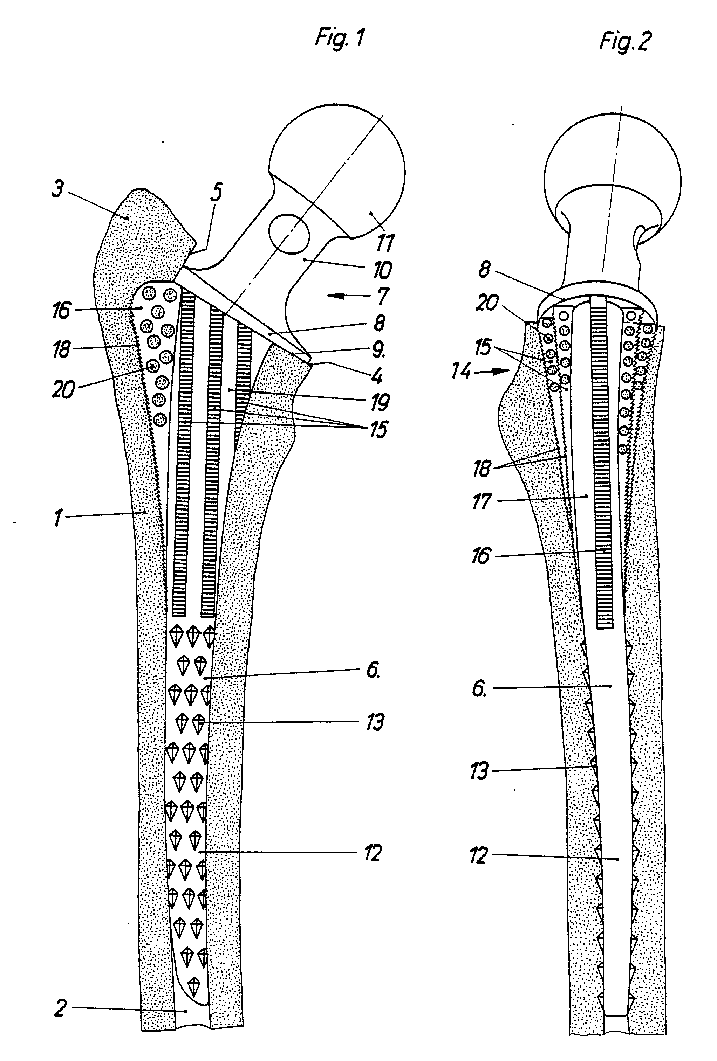

Die Schnittansichtsflächen des Oberschenkelknochens 1 sind in der Zeichnung punktiert angelegt. Man erkennt die Markhöhle 2, in Fig. 1 lateral dem großen Trochanter 3 sowie die Resektionsflächen 4, 5, längs welchen der Oberschenkelhals mit dem Gelenkkopf entfernt ist.

In der ausgeräumten Markhöhle 2 befindet sich der zementlos eingesetzte Schaft 6 der Prothese 7. Der Schaft 6 wird proximal durch die Halsauflage 8 abgeschlossen, deren Unterfläche 9 auf der Resektionsfläche 4 aufliegt. Es schließt sich der Hals 10 mit dem Gelenkkopf 11 an. In den Ausführungsbeispielen der Fig. 1 bis 3 ist vorausgesetzt, daß alle diese Prothesenteile einstückig aus demselben Material, beispielsweise geschmiedetem Titan, bestehen.

Der Prothesenschaft 6 ist in seinem unteren Bereich 12 mit geschlossener Oberfläche ausgeführt, die zwecks besseren Verbundes mit dem Knochen mit Noppen 13 irgendeiner geeigneten Form versehen sein kann. In diesem Bereich ist die Markhöhle des Knochens vergleichsweise eng und individuell weniger unterschiedlich, so daß keine große Zahl variierender Prothesenformen erforderlich ist.

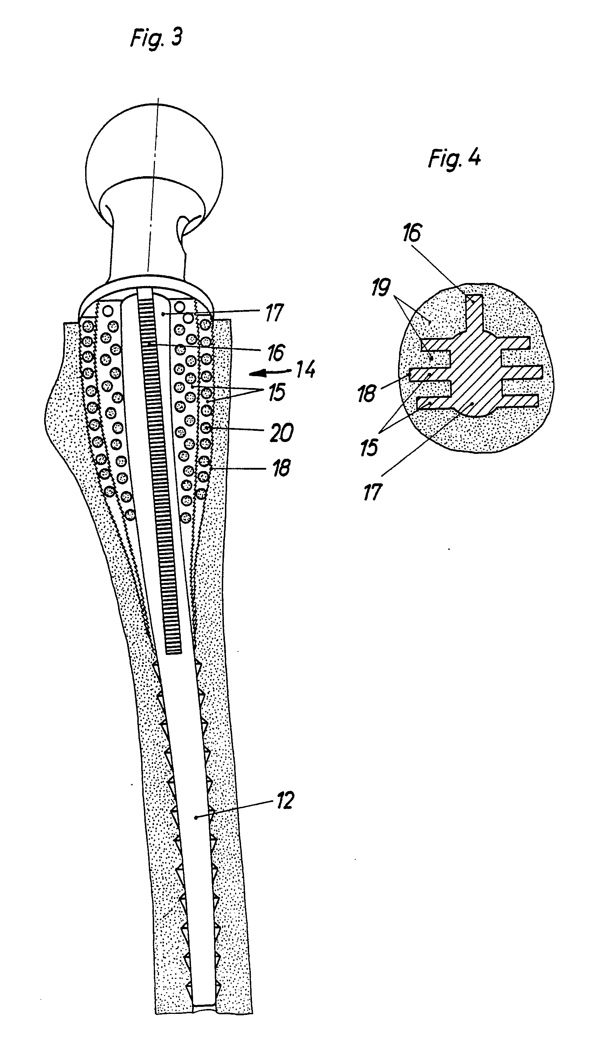

Am proximalen Ende erweitert sich die Markhöhle 2 in dem Bereich, der allgemein mit der Bezugsziffer 14 angedeutet ist, im allgemeinen trompetenartig wie dies in den Fig. 1 und 2 vorausgesetzt wurde. Es kommen jedoch auch andere Erweiterungsformen vor, wie dies beispielsweise in Fig. 3 gezeigt ist. Um der Notwendigkeit zu entgehen, den Schaft 6 in diesem erweiterten Bereich massiv und in einer großen Zahl von Formvariationen entsprechend der individuellen Innengestalt der Corticalis zu gestalten, weist der Prothesenschaft eine Mehrzahl von Rippen 15, 16 auf, die von dem vergleichsweise dünnen Schaftkern 17 im Querschnitt weit nach außen vordringen und sich teilweise der Corticalis nähern. Zumindest füllt der von den Rippen bestimmte Prothesenquerschnitt den spongiösen Teil des trochanteren Femurbereichs großenteils aus. Die Tiefe der Rippen (Hauptabmessung von ihrer Rückenfläche 18 bis zum Schaftkern 17) ist am proximalen Ende am größten, während sie distal allmählich bis auf Null verschwindet.

Im Querschnitt (Fig. 4) sind in den gegebenen Beispielen sechs Rippen 15 auf gegenüberliegenden Seiten parallel zueinander angeordnet, während eine Rippe 16 quer dazu verläuft. Diese Anordnung gestattet die Herstellung durch Schmieden. Wenn ein anderes Herstellungsverfahren gewählt wird, können die Rippen auch in anderer Anordnung verlaufen. Wichtig ist jedoch stets, daß zumindest die am weitesten medial gelegenen Rippen ausgeprägte, nach medial gerichtete Kraftübertragungsflächen bilden. In einem wesentlichen Teil ihrer Länge ist ihre Tiefenabmessung größer als ihre Breite oder wenigstens etwa dieser gleich. Zwischen den Rippen 15, 16 befinden sich Zwischenräume 19, in welchen das vorhandene Knochengewebe verbleiben oder neues sich bilden kann. Die Rippen sind mit einer Vielzahl von Querbohrungen 20 versehen, die die Widerstandskraft der Rippen gegenüber Längskräften herabsetzen und diese dadurch nachgiebiger machen. Dieser Effekt wird in denjenigen Bereichen, in welchen die Rippen besonders tief sind, durch versetzte Anordnung der Durchbrechungen verstärkt. Außerdem kann Knochengewebe in die Durchbrechungen hineinwachsen und dadurch die Verankerung des Prothesenschafts im Knochen verbessern und auch die Übertragung von Zugkräften gestatten.

Die Rückenflächen 18 der Rippen sind gezahnt, was nicht nur dem formschlüssigen Verbund mit dem Knochengewebe dienen soll, sondern beim Einsetzen in den Röhrenknochen einen der Arbeitsweise einer Räumnadel gleichenden Effekt haben kann, durch den bei entsprechend knapper Vorbearbeitung des Knochenhohlraums gewährleistet wird, daß die Rückenflächen der Rippen ohne Zwischenraum am Knochengewebe anliegen.

Die Rippen können entsprechend der Richtung der Einsetzbewegung des Prothesenschafts in den Knochen gekrümmt sein, damit möglichst wenig Knochensubstanz vor dem Einsetzen bzw.

während desselben weggeräumt werden muß.

Durch die ausgedehnten, mit dem Knochengewebe zusammenwirkende Seitenflanken der Rippen 15 wird die Kraftübertragung nach medial begünstigt. Die laterale Rippe 16, die in bekannter Anordnung in dem Trochanter eingreift, beteiligt auch diesen an der Kraftübertragung. Zusätzlich können weitere Mittel zur Übertragung von Kräften vom Prothesenschaft oder von der Halsauflage 8 auf die lateralen Bereiche des Knochens vorgesehen sein.

Man erkennt am Vergleich der Ausführungsbeispiele der Fig. 2 und 3, daß Prothesen mit unterschiedlicher Rippenform aus gleichen Rohlingen durch entsprechend unterschiedliches Abfräsen der Rippenrücken erzeugt werden können.

Die mittlere Weite der Zwischenräume sollte im allgemeinen nicht kleiner als 2 mm sein. Als zweckmäßig hat sich eine mittlere Weite von etwa 3 mm erwiesen.

## Patentanspruch

Verfahren zum Herstellen von Endoprothesen mit in die Markhöhle (2) von Knochen (1) einzusetzenden Halteschäften (6) unterschiedlicher Gestalt, bei dem die Oberflächen von Rohlingen gleicher Gestalt unterschiedlich bearbeitet werden, dadurch gekennzeichnet, daß die Rohlinge mit von einem Schaftkern (17) vorstehenden Rippen (15, 16) hergestellt und die Rippenrücken (18) bearbeitet werden.

## Claim

A method of producing endoprostheses with support shafts (6) of different shape to be inserted in the medullary cavity (2) of a bone (1), wherein the surfaces of blanks of like shape are differently machined, characterised in that the blanks are produced with ribs (15, 16) projecting from a shaft core (17) and the rib backs (8) are machined.

## Revendication

Procédé de fabrication d'endoprothèses comportant des tiges de maintien (6), de configurations différentes, destinées à être insérées dans la cavité médullaire (2) d'os (1), les surfaces d'ébauches ayant la même configuration étant usinées différemment, caractérisé en ce que les ébauches sont fabriquées avec des nervures (15, 16) faisant saillie sur une âme de tige (17) et en ce que les dos (18) des nervures sont usinés.

Fig. 1

Fig. 2

*Fig. 3*

*Fig. 4*

17
16
14
15
20
18

16
19
18
15
17

12

2